# EUROPEAN PATENT APPLICATION

(11) **EP 4 197 532 A1**
(43) Date of publication of application: **21.06.2023**
(21) Application number: 21855631.4
(22) Date of filing: 13.08.2021
(51) Int. Cl.: A61K 31/138, A61P 17/08, A61P 17/02

(54) **METHOD FOR TREATING DERMATITIS**

(30) Priority: 14.08.2020 US 202063065723 P
(71) Applicant: Chang Gung Memorial Hospital, Linkou, Taoyuan City 33305 (TW)
(72) Inventor: LU, Chun-Wei, Fuxin (CN); CHUNG, Wen-Hung, Fuxin (CN); KO, Yu-Shien, Fuxin (CN); SU PANG, Jong-Hwei, Taiwan 33302 (CN)
(74) Representative: Cabinet Becker et Associés
(86) International application number: PCT/CN2021/112493
(87) International publication number: WO 2022/033581

(57) **Abstract**

The present invention relates to methods for treating dermatitis and the use of a β-1 adrenoceptor antagonist in manufacturing a pharmaceutical composition for treating dermatitis. The methods comprise the step of administering the pharmaceutical composition comprising a therapeutically effective amount of β-1 adrenoceptor antagonist to a subject in need thereof.

## Description

### Technical Field

### Cross-references to related applications

This application claims the benefit of priority from US Provisional Application No. 63/065,723, filed on August 14, 2020. The entire contents of the above application are incorporated herein by reference.

The present invention relates to a method for treating dermatitis and the use of a β-1 adrenergic receptor antagonist for manufacturing a pharmaceutical composition for treating dermatitis.

### Background

Dermatitis is a broad term that covers a range of inflammatory skin conditions with different etiologies; common dermatitis symptoms include erythema, scaling, crusting, vesicles, itching, and skin thickening due to chronic inflammation.

The main treatment for dermatitis focuses on basic skin care with moisturizers and topical steroid and immunosuppressant treatments. However, long-term use of topical steroid and immunosuppressant for dermatitis can lead to adverse consequences, such as skin thinning and prone to infection, as well as skin burning/irritation and increased risk of skin cancer. In addition, some patients with dermatitis do not respond to these treatments (i.e., they are resistant to steroids and immunosuppressants) and continue to suffer from extensive skin lesions and intense itching, resulting in physical and mental disability.

In conclusion, there is an unmet need for the treatment of dermatitis, and the present invention addresses this and other needs.

### SUMMARY OF THE INVENTION

The present invention provides a method of treating dermatitis.

The present invention provides the use of β-1 adrenergic receptor antagonists for manufacturing pharmaceutical compositions for treating dermatitis.

In one embodiment, the present invention discloses a method of treating dermatitis, comprising the step of administering to a subject in need thereof a pharmaceutical composition comprising a therapeutically effective amount of a β-1 adrenergic receptor antagonist.

In one embodiment, the present invention discloses the use of a β-1 adrenergic receptor antagonist in manufacturing a pharmaceutical composition for treating dermatitis, comprising the step of administering a therapeutically effective amount of β-1 adrenergic receptor antagonist to a patient in need thereof.

The terms "the invention," "this invention," and "the present invention" as used in this patent refer broadly to all of the subject matters of this patent and the claims that follow. The recitations of these terms should be construed as not limiting the meaning of the subject matter described or the following claims.

Embodiments of the invention covered by this patent are defined by the following claims rather than the summary of the invention. This summary of invention is a high-level overview of various aspects of the invention and introduces some of the concepts further described in the Implementation section below. This summary is not intended to define key or essential technical features of the claimed subject matter, nor is it intended to be used alone to define the scope of the claimed subject matter, but should be referenced to appropriate paragraphs throughout the specification, any or all drawings and each claim to understand the subject matter.

The present invention will become more apparent when read in conjunction with the accompanying drawings and the following detailed description.

### Description of drawings

Illustrative embodiments of the present invention have been described in detail with reference to the accompanying drawings.
Figure 1(A) is a photographic image of a patient with eczema who did not respond to high-dose steroid, and Figure 1(B) is a photographic image of the patient with eczema treated with topical betaxolol for 2 days.
Figure 2(A) to Figure 2(C) are photographic images showing the efficacy of topical betaxolol in treating a patient with eczema induced by epidermal growth factor receptor tyrosine kinase inhibitor (osimertinib), on day 0 (Figure 2(A)), day 2 (Figure 2(B)) and day 7 (Figure 2(C)).
Figure 3(A) and Figure 3(B) are photographic images showing a patient with poorly controlled dermatitis before treatment (Figure 3(A)) and after 2 days of topical betaxolol treatment to the affected area (Figure 3(B)).
Figure 4(A) and Figure 4(B) are photographic images showing a patient with poorly controlled dermatitis before treatment (Figure 4(A)) and after 4 weeks of topical betaxolol treatment (Figure 4(B)).
Figure 5(A) and Figure 5(B) are photographic images showing a patient with poorly controlled dermatitis before treatment (Figure 5(A)) and after 7 days of topical betaxolol treatment (Figure 5(B)).

### Implementation

The following content, in combination with the accompanying drawings, illustrate the technical content of the present invention through specific embodiments. Those skilled in the art can easily understand other advantages and effects of the present invention from the content disclosed in this specification. The present invention can also be implemented or applied through other different specific embodiments. Various details in this specification can also be modified and changed based on different viewpoints without departing from the spirit of the present invention.

As used herein, the term "a" and "an" refer to one or more (i.e., at least one) grammatical obj ects.

The terms "individual" and "patient" are used interchangeably and refer to mammals diagnosed with, suspected of having, susceptible to, or in need of prevention of dermatitis, the latter including those patients about to undergo epidermal growth factor receptor tyrosine kinase inhibitor (EGFR-TKI) treatment for cancer; the subject or patient includes a primate, preferably a human.

An "effective amount" of an antagonist is one that produces a desired effect compared to an untreated subject; for example, an amount of a β-1 adrenergic receptor antagonist that reduces symptoms and signs of dermatitis by at least 1%.

As used herein, the term "treatment" refers to both therapeutic treatment and prophylactic or preventive measures; persons in need of treatment include those already suffering from dermatitis, as well as those susceptible to dermatitis or those in need of prophylaxis.

All numbers herein are understood to be modified by "about". As used herein, the term "about" is intended to encompass a variation of ±10%.

The present invention relates to the use of a β-1 adrenergic receptor antagonist in manufacturing a pharmaceutical composition for treating dermatitis, including the step of administering a pharmaceutical composition comprising a therapeutically effective amount of the β-1 adrenergic receptor antagonist to a patient in need thereof.

As used herein, dermatitis is any form of skin inflammation and usually involves itching, dry skin, or a rash on swollen and erythematous skin. Other signs and symptoms of dermatitis include blisters, exudates, crusts or skin flaking. Non-limiting examples of skin cancer include atopic dermatitis (eczema), targeted therapy-induced dermatitis, steroid-resistant dermatitis, contact dermatitis, seborrheic dermatitis (e.g., dandruff), and seborrheic dermatitis.

As used herein, targeted therapy includes therapeutic agent that inhibits cancer cell growth by interfering with specific targeted molecules required for cancer development and cancer growth, rather than simply by interfering with rapidly dividing cells (e.g., conventional chemotherapy), such as kinase inhibitors, angiogenesis inhibitors, epidermal growth factor receptor (EGFR) inhibitors (e.g., epidermal growth factor receptor tyrosine kinase inhibitor, EGFR-TKI), HER2/neu receptors, or any combination thereof.

In some embodiments, the dermatitis is substantially free of cracks or erosions.

In some embodiments, the pharmaceutical composition comprises a β-1 adrenergic receptor antagonist. In other embodiments, the pharmaceutical composition is substantially free of a β-2 adrenergic receptor antagonist, a non-selective β adrenergic receptor antagonist, antibodies (e.g., antibodies against IL4RA or IL-25), or any combination thereof.

B-1 adrenergic receptor antagonists can be administered with a pharmaceutically acceptable carrier.

Pharmaceutical compositions can be formulated for systemic (e.g., oral or intravenous), intradermal, subcutaneous, intramuscular, or topical administration. In some embodiments, the pharmaceutical composition can be formulated for topical delivery in one of the following forms: ointment, cream, solution, gel, suspension, spray, or lotion. In other embodiments, the pharmaceutical combination may be formulated for slow release or sustained release.

Non-limiting examples of β-1 adrenergic receptor antagonist include atenolol, betaxolol, bisoprolol, esmolol, acebutolol, metoprolol, nebivolol, or any combination thereof.

In one embodiment, the pharmaceutical composition manufactured for treating dermatitis further comprises at least one therapeutic agent effective for dermatitis; such therapeutic agents include but are not limited to steroids (e.g. prednisolone, methylprednoslone, betamethasone, dexamethasone, clobetasole, etc.), anti-histamines (e.g. levocetirizine, dexchlorpheniramine, fexofenadine, desloratadine, hydroxyzine, etc.), immunomodulators (such as azathioprine, methotrexate, cyclosporine, tacrolimus or pimecrolimus), antibiotics (e.g. fusidic acid) or light therapy. In certain embodiments, the above-mentioned therapeutic agents for the treatment of dermatitis may be used in combination with a pharmaceutical composition comprising a β-1 adrenergic receptor antagonist.

When a pharmaceutical composition comprising a β-1 adrenergic receptor antagonist is administered in combination or alternating with at least one therapeutic agent for treating dermatitis, the β-1 adrenergic receptor antagonist in the pharmaceutical composition may be administered at a lower dosage, less frequent dosing and/or increased dosing intervals to achieve a therapeutic effect.

In addition, one skilled in the art can readily determine the appropriate dosage of a pharmaceutical composition comprising a β-1 adrenergic receptor antagonist to be administered for a particular type of dermatitis; e.g., the severity and type of dermatitis, the patient's age, weight, gender, comorbidities, and other co-administered therapeutic agents.

Those skilled in the art will recognize that the preferred dose is that which produces a therapeutic effect, such as reduction of symptoms and signs of dermatitis, in a patient in need thereof. In certain embodiments, one dose is administered daily for a specified number of days (e.g., 7 days, 1 month, etc.). In other embodiments, multiple doses may be administered within a day (every 2, 4, 6, or 12 hours, etc.). The present invention also contemplates multiple administrations over multiple days.

Embodiments of the present invention are illustrated by the following examples, which should not be construed in any way to limit its scope. On the contrary, it should be clearly understood that after reading the contents herein, those skilled in the art may resort to various embodiments, modifications and their equivalents without departing from the spirit of the present invention. In the experiments described in the following examples, unless otherwise stated, routine experimental procedures were followed. Some experimental procedures are described for illustrative purpose.

### Example 1

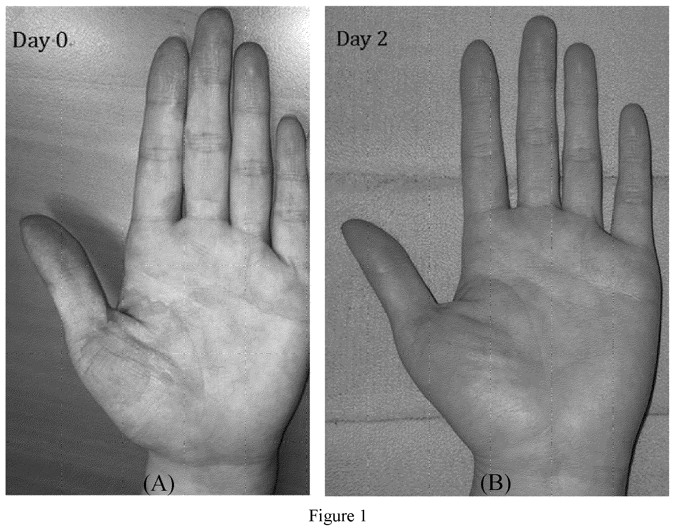

A patient with hand eczema had been treated with high-dose topical steroid (clobetasol ointment twice a day for a week) without any improvement, and presented with erythema and scaling (see Figure 1(A). The patient was treated with 0.25% (w/w) betaxolol gel twice a day to the affected area and after 2 days, the erythema and scaling subsided significantly (see Figure 1(B)).

### Example 2

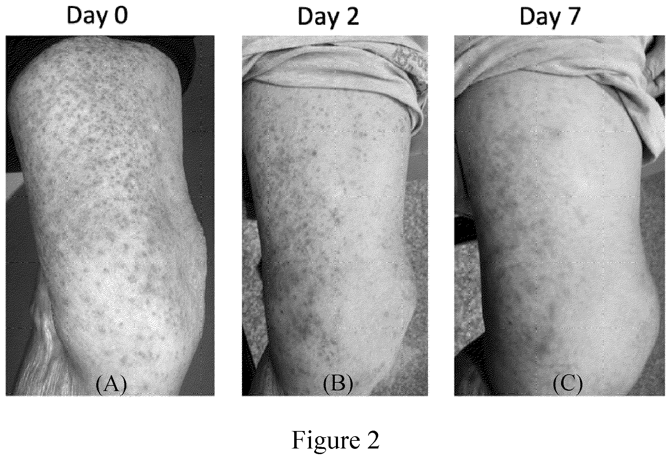

A patient with generalized dermatitis (eczema) induced by an epidermal growth factor receptor inhibitor (osimertinib), was treated with steroid (methylprednisolone (20 mg) twice a day for 21 days) but did not improve (refer to Figure 2(A)). The patient was subsequently treated with betaxolol gel twice a day, and the dermatitis significantly improved after 2 days and 7 days of treatment. (see Figure 2(B) and Figure 2(C)).

### Example 3

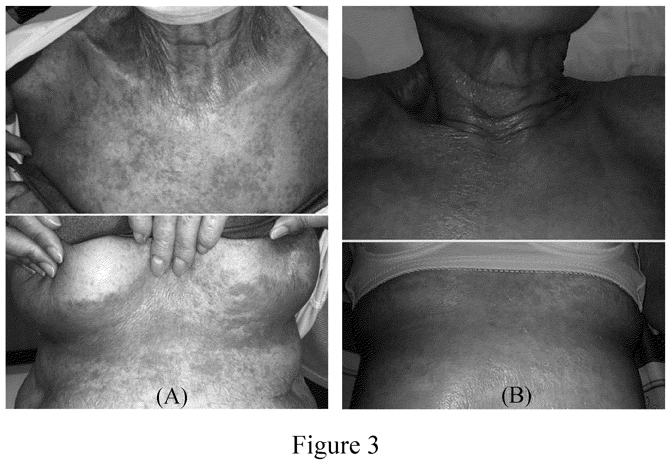

A patient with generalized dermatitis was poorly controlled despite high-dose oral steroid treatment (methylprednisolone (20 mg) twice a day for 14 days) and presented with a generalized erythematous rash (see Figure 3(A)). The patient's was then treated with 0.25% (w/w) betaxolol gel twice a day to the affected area and after 2 days, the erythema rash completely resolved (refer to Figure 3(B)).

### Example 4

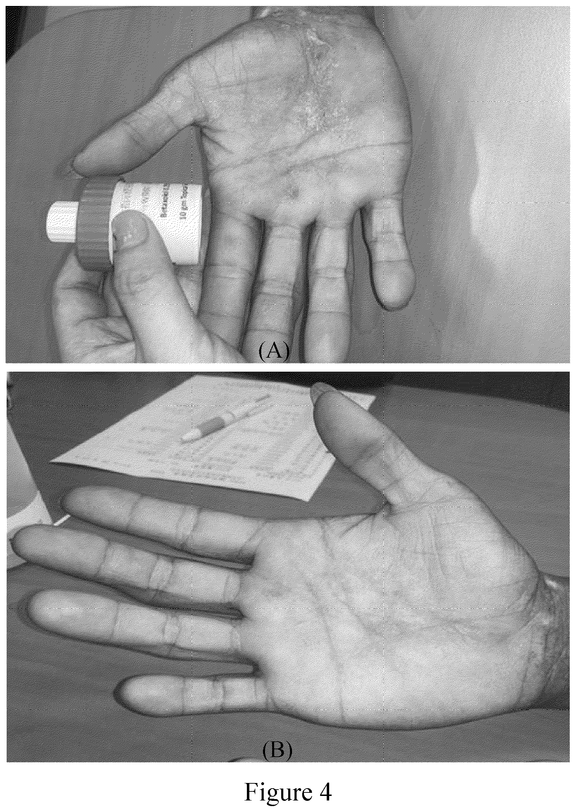

A middle-aged male patient with dermatitis in the hands was given topical steroid ointment (clobetasol ointment (0.025%) twice a day for 21 days), oral anti-histamines (desloratadine (5 mg) once a day + hydroxyzine (25 mg) twice a day for 21 days) and immunosuppressant (cyclosporine (100 mg) twice a day for 28 days), but the scaling and chronic inflammation did not improve (see Figure 4(A)). This patient was subsequently treated with 0.25% (w/w) betaxolol gel twice daily to the affected area for 4 weeks, and scaling and inflammation completely subsided (refer to Figure 4(B)).

### Example 5

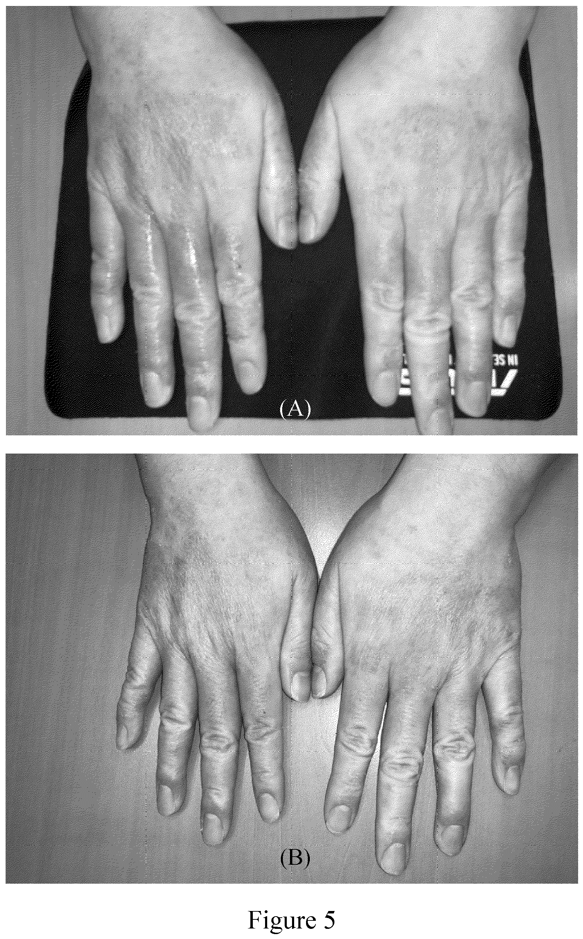

A patient with hand dermatitis without any treatment (see Figure 5(A)) was treated with 0.25% (w/w) betaxolol gel topical to affected area twice a day for 7 days. The erythema and inflammation significantly improved after 2 days of treatment (see Figure 5(B)).

In conclusion, β-1 adrenergic receptor antagonists, compared with conventional therapeutic agents such as general steroids, antihistamines and immunosuppressants, do have obvious therapeutic ability for dermatitis and have unpredictable effects; although The examples use only betaxolol as a specific β-1 adrenergic receptor antagonist, but it is only an exemplary β-1 adrenergic receptor antagonist and is not thereby limited to β-1 adrenergic receptor antagonists Only betaxolol has the effect of treating dermatitis.

The above-mentioned examples merely illustrate the principle and effect of the present invention, but are not intended to limit the present invention. Anyone skilled in the art can modify and change the above embodiments without departing from the spirit and scope of the present invention. Therefore, the protection scope of the present invention should be as listed in the claims of the present invention.

## Claims

1. Use of a β-1 adrenergic receptor antagonist in manufacturing a pharmaceutical composition for treating dermatitis, comprising the step of administering to a subject in need thereof the pharmaceutical composition comprising a therapeutically effective amount of the β-1 adrenergic receptor antagonist.

2. The use according to claim 1, wherein the dermatitis is atopic dermatitis (eczema).

3. The use according to claim 1, wherein the dermatitis is induced by an epidermal growth factor receptor tyrosine kinase inhibitor (EGFR-TKI).

4. The use according to claim 1, wherein the dermatitis is steroid-resistant

5. The use according to claim 1, wherein the skin cancer is seborrheic dermatitis.

6. The use according to claim 1, wherein the β-1 adrenergic receptor antagonist is selected from atenolol, betaxolol, bisoprolol, esmolol, acebutolol, metoprolol, nebivolol, or any combination thereof.

7. The use according to claim 1, further comprising the step of administering steroid, anti-histamine, immunomodulator, antibiotic, light therapy, or any combination thereof.

8. The use according to claim 7, wherein the immunomodulator is cyclosporine, tacrolimus or pimecrolimus.

9. The use according to claim 1, wherein the pharmaceutical composition is substantially free of an antibody.

10. The use of claim 1, wherein the antibody is an antibody against IL4RA or IL-25.
